# EUROPEAN PATENT SPECIFICATION

(11) **EP 4 090 279 B1**
(45) Date of publication and mention of the grant of the patent: **04.06.2025**
(21) Application number: 21841022.3
(22) Date of filing: 29.12.2021
(51) Int. Cl.: A61B 18/14, A61B 17/32, A61B 17/00, A61B 18/00, A61B 18/16, A61B 18/12

(54) **ELECTROSURGICAL INSTRUMENT WITH SHAFT VOLTAGE MONITOR**
ELEKTROCHIRURGISCHES INSTRUMENT MIT WELLENSPANNUNGSÜBERWACHUNG
INSTRUMENT ÉLECTROCHIRURGICAL AVEC DISPOSITIF DE SURVEILLANCE DE TENSION D'ARBRE

(30) Priority: 29.12.2020 US 202017136145
(43) Date of publication of application: 23.11.2022
(73) Proprietor: Cilag GmbH International, 6300 Zug (CH)
(72) Inventor: SHELTON, IV, Frederick E., Cincinnati, Ohio 45242 (US); YATES, David C., West Chester, Ohio 45069 (US)
(74) Representative: Carpmaels & Ransford LLP
(86) International application number: PCT/IB2021/062413
(87) International publication number: WO 2022/144789

(56) References cited:
- US-A- 5 312 401
- US-A1- 2006 041 252
- US-A1- 2010 042 097

## Description

### BACKGROUND

A variety of ultrasonic surgical instruments include an end effector having a blade element that vibrates at ultrasonic frequencies to cut and/or seal tissue (e.g., by denaturing proteins in tissue cells). These instruments include one or more piezoelectric elements that convert electrical power into ultrasonic vibrations, which are communicated along an acoustic waveguide to the blade element. Examples of ultrasonic surgical instruments and related concepts are disclosed in U.S. Pub. No. 2006/0079874, entitled "Tissue Pad for Use with an Ultrasonic Surgical Instrument," published April 13, 2006, now abandoned; U.S. Pub. No. 2007/0191713, entitled "Ultrasonic Device for Cutting and Coagulating," published August 16, 2007; and U.S. Pub. No. 2008/0200940, entitled "Ultrasonic Device for Cutting and Coagulating," published August 21, 2008, now abandoned.

Some instruments are operable to seal tissue by applying radiofrequency (RF) electrosurgical energy to the tissue. Examples of such devices and related concepts are disclosed in U.S. Pat. No. 7,354,440, entitled "Electrosurgical Instrument and Method of Use," issued April 8, 2008; U.S. Pat. No. 7,381,209, entitled "Electrosurgical Instrument," issued June 3, 2008.

Some instruments are capable of applying both ultrasonic energy and RF electrosurgical energy to tissue. Examples of such instruments are described in U. S. Patent No. 9,949,785, entitled "Ultrasonic Surgical Instrument with Electrosurgical Feature," issued April 24, 2018; and U.S. Patent No. 8,663,220, entitled "Ultrasonic Electrosurgical Instruments," issued March 4, 2014.

In some scenarios, it may be preferable to have surgical instruments grasped and manipulated directly by the hand or hands of one or more human operators. In addition, or as an alternative, it may be preferable to have surgical instruments controlled via a robotic surgical system. Examples of robotic surgical systems and associated instrumentation are disclosed in U.S. Pat. No. 10,624,709, entitled "Robotic Surgical Tool with Manual Release Lever," published on May 2, 2019; U.S. Pat. No. 9,314,308, entitled "Robotic Ultrasonic Surgical Device With Articulating End Effector," issued on April 19, 2016; U.S. Pat. No. 9,125,662, entitled "Multi-Axis Articulating and Rotating Surgical Tools," issued September 8, 2015; U.S. Pat. No. 8,820,605, entitled "Robotically-Controlled Surgical Instruments," issued Sep. 2, 2014; U.S. Pub. No. 2019/0201077, entitled "Interruption of Energy Due to Inadvertent Capacitive Coupling," published July 4, 2019; U.S. Pub. No. 2012/0292367, entitled "Robotically-Controlled End Effector," published on November 11, 2012, now abandoned; and U.S. Pat. App. No. 16/556,661, entitled "Ultrasonic Surgical Instrument with a Multi-Planar Articulating Shaft Assembly," filed on August 30, 2019.

US 2006/0041252 Al discloses a method of applying an active electrode to a patient and placing a return electrode on the patient so as to create a current path in tissue of the patient between the active electrode and the return electrode. A conductive element, which is operatively coupled to the active electrode, is coupled to a reference voltage with a low impedance path and a voltage is imparted to the active electrode so as to generate current in the current path. Any undesirable current flow that would otherwise flow from the active electrode to the reference voltage through the patient is limited to reduce a risk of harm to the patient.

### SUMMARY OF INVENTION

The invention is defined in the claims.

### BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements and in which:
FIG. 1 depicts a schematic view of an example of a robotic surgical system;
FIG. 2 depicts a schematic view of an example of a robotic surgical system being used in relation to a patient;
FIG. 3 depicts a schematic view of examples of components that may be incorporated into a surgical instrument;
FIG. 4 depicts a side elevation view of an example of a handheld surgical instrument;
FIG. 5 depicts a perspective view of an example of an end effector that is operable to apply ultrasonic energy to tissue;
FIG. 6 depicts a perspective view of an example of an end effector that is operable to apply bipolar RF energy to tissue;
FIG. 7 depicts a schematic view of an example of a surgical instrument that is operable to apply monopolar RF energy to tissue;
FIG. 8 depicts a perspective view of an example of an articulation section that may be incorporated into a shaft assembly of a surgical instrument;
FIG. 9 depicts a side elevation view of a portion of a shaft assembly that may be incorporated into a surgical instrument, with housing components of the shaft being shown in cross-section to reveal internal components of the shaft;
FIG. 10 depicts a cross-sectional end view of another shaft assembly that may be incorporated into a surgical instrument;
FIG. 11 depicts a schematic view of a portion of another shaft assembly that may be incorporated into a surgical instrument;
FIG. 12 depicts a perspective view of an example of a surgical instrument that may be incorporated into the robotic surgical system of FIG. 1;
FIG. 13 depicts a top plan view of an interface drive assembly of the instrument of FIG. 12;
FIG. 14 depicts a cross-sectional side view of an articulation section of a shaft assembly of the instrument of FIG. 12;
FIG. 15 depicts a perspective view of another example of a handheld surgical instrument, with a modular shaft assembly separated from a handle assembly; and
FIG. 16 depicts a side elevation view of a portion of another shaft assembly that may be incorporated into a surgical instrument, with housing components of the shaft being shown in cross-section to reveal internal components of the shaft.

The drawings are not intended to be limiting in any way, and it is contemplated that various embodiments of the technology may be carried out in a variety of other ways, including those not necessarily depicted in the drawings. The accompanying drawings incorporated in and forming a part of the specification illustrate several aspects of the present technology, and together with the description explain the principles of the technology; it being understood, however, that this technology is not limited to the precise arrangements shown.

### DETAILED DESCRIPTION

The following description of certain examples of the technology should not be used to limit its scope. Other examples, features, aspects, embodiments, and advantages of the technology will become apparent to those skilled in the art from the following description, which is by way of illustration, one of the best modes contemplated for carrying out the technology. As will be realized, the technology described herein is capable of other different and obvious aspects, all without departing from the technology. Accordingly, the drawings and descriptions should be regarded as illustrative in nature and not restrictive.

It is further understood that any one or more of the teachings, expressions, embodiments, examples, etc. described herein may be combined with any one or more of the other teachings, expressions, embodiments, examples, etc. that are described herein. The following-described teachings, expressions, embodiments, examples, etc. should therefore not be viewed in isolation relative to each other. Various suitable ways in which the teachings herein may be combined will be readily apparent to those of ordinary skill in the art in view of the teachings herein. Such modifications and variations are intended to be included within the scope of the claims.

For clarity of disclosure, the terms "proximal" and "distal" are defined herein relative to a human or robotic operator of the surgical instrument. The term "proximal" refers the position of an element closer to the human or robotic operator of the surgical instrument and further away from the surgical end effector of the surgical instrument. The term "distal" refers to the position of an element closer to the surgical end effector of the surgical instrument and further away from the human or robotic operator of the surgical instrument. In addition, the terms "upper," "lower," "top," "bottom," "above," and "below," are used with respect to the examples and associated figures and are not intended to unnecessarily limit the invention described herein.

### I. Example of a Robotic Surgical System

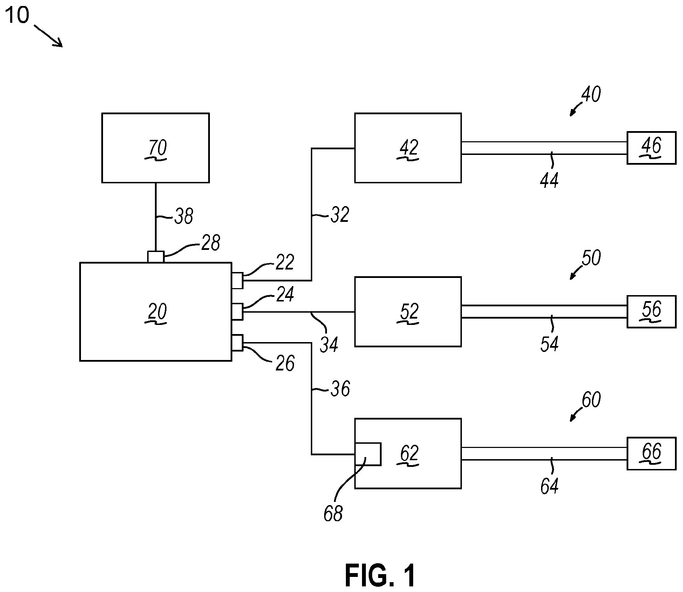

As noted above, in some surgical procedures, it may be desirable to utilize a robotically controlled surgical system. Such a robotically controlled surgical system may include one or more surgical instruments that are controlled and driven robotically via one or more users that are either in the same operating room or remote from the operating room. FIG. 1 illustrates on example of various components that may be incorporated into a robotic surgical system (10). System (10) of this example includes a console (20), a monopolar RF electrosurgical instrument (40), a bipolar RF electrosurgical instrument (50), and an ultrasonic surgical instrument (60). While FIG. 1 shows all three instruments (40, 50, 60) coupled with console (20) at the same time, there may be usage scenarios where only one or two of instruments (40, 50, 60) coupled with console (20) at the same time. In addition, there may be usage scenarios where various other instruments are coupled with console (20) in addition, or as an alternative to, one or more of instruments (40, 50, 60) being coupled with console (20).

Monopolar RF electrosurgical instrument (40) of the present example includes a body (42), a shaft (44) extending distally from body (42), and an end effector (46) at the distal end of shaft (44). Body (42) is configured to couple with a robotic arm (not shown in FIG. 1) of system (10), such that the robotic arm is operable to position and orient monopolar RF electrosurgical instrument (40) in relation to a patient. In versions where monopolar RF electrosurgical instrument (40) includes one or more mechanically driven components (e.g., jaws at end effector (46), articulating sections of shaft (44), rotating sections of shaft (44), etc.), body (42) may include various components that are operable to convert one or more mechanical drive inputs from the robotic arm into motion of the one or more mechanically driven components of monopolar RF electrosurgical instrument (40).

As also shown in FIG. 1, body (42) is coupled with a corresponding port (22) of console (20) via a cable (32). Console (20) is operable to provide electrical power to monopolar RF electrosurgical instrument (40) via port (22) and cable (32). In some versions, port (22) is dedicated to driving monopolar RF electrosurgical instruments like monopolar RF electrosurgical instrument (40). In some other versions, port (22) is operable to drive various kinds of instruments (e.g., including instruments (50, 60), etc.). In some such versions, console (20) is operable to automatically detect the kind of instrument (40, 50, 60) that is coupled with port (22) and adjust the power profile to port (22) accordingly. In addition, or in the alternative, console (20) may adjust the power profile to port (22) based on a selection made by an operator via console (20), manually identifying the kind of instrument (40, 50, 60) that is coupled with port (22).

Shaft (44) is operable to support end effector (46) and provides one or more wires or other paths for electrical communication between base (42) and end effector (46). Shaft (44) is thus operable to transmit electrical power from console (20) to end effector (46). Shaft (44) may also include various kinds of mechanically movable components, including but not limited to rotating segments, articulating sections, and/or other kinds of mechanically movable components as will be apparent to those skilled in the art in view of the teachings herein.

End effector (46) of the present example includes an electrode that is operable to apply monopolar RF energy to tissue. Such an electrode may be incorporated into a sharp blade, a needle, a flat surface, some other atraumatic structure, or any other suitable kind of structure as will be apparent to those skilled in the art in view of the teachings herein. End effector (46) may also include various other kinds of components, including but not limited to grasping jaws, etc.

System (10) of this example further includes a ground pad (70) that is coupled with a corresponding port (28) of console (20) via a cable (38). In some versions, ground pad (70) is incorporated into a patch or other structure that is adhered to the skin of the patient (e.g., on the thigh of the patient). In some other versions, ground pad (70) is placed under the patient (e.g., between the patient and the operating table). In either case, ground pad (70) may serve as a return path for monopolar RF energy that is applied to the patient via end effector (46). In some versions, port (28) is a dedicated ground return port. In some other versions, port (28) is a multi-purpose port that is either automatically designated as a ground return port upon console (20) detecting the coupling of ground pad (70) with port (28) or manually designated as a ground return port via an operator using a user input feature of console (20).

Bipolar RF electrosurgical instrument (50) of the present example includes a body (52), a shaft (54) extending distally from body (52), and an end effector (56) at the distal end of shaft (54). Each of these components (52, 54, 56) may be configured and operable in accordance with the above description of corresponding components (42, 44, 46) of monopolar RF electrosurgical instrument (50), except that end effector (56) of this example is operable to apply bipolar RF energy to tissue. Thus, end effector (56) includes at least two electrodes, with those two electrodes being configured to cooperate with each other to apply bipolar RF energy to tissue. Bipolar RF electrosurgical instrument (50) is coupled with console (20) via a cable (34), which is further coupled with a port (24) of console (20). Port (24) may be dedicated to powering bipolar RF electrosurgical instruments. Alternatively, port (24) or may be a multi-purpose port whose output is determined based on either automatic detection of bipolar RF electrosurgical instrument (50) or operator selection via a user input feature of console (20).

Ultrasonic surgical instrument (60) of the present example includes a body (62), a shaft (64) extending distally from body (62), and an end effector (66) at the distal end of shaft (64). Each of these components (62, 64, 66) may be configured and operable in accordance with the above description of corresponding components (42, 44, 46) of monopolar RF electrosurgical instrument (50), except that end effector (66) of this example is operable to apply ultrasonic energy to tissue. Thus, end effector (66) includes an ultrasonic blade or other ultrasonically vibrating element. In addition, base (62) includes an ultrasonic transducer (68) that is operable to generate ultrasonic vibrations in response to electrical power, while shaft (64) includes an acoustic waveguide that is operable to communicate the ultrasonic vibrations from transducer (68) to end effector (66).

Ultrasonic surgical instrument (60) is coupled with console (20) via a cable (36), which is further coupled with a port (26) of console (20). Port (26) may be dedicated to powering ultrasonic electrosurgical instruments. Alternatively, port (26) or may be a multi-purpose port whose output is determined based on either automatic detection of ultrasonic instrument (60) or operator selection via a user input feature of console (20).

While FIG. 1 shows monopolar RF, bipolar RF, and ultrasonic capabilities being provided via three separate, dedicated instruments (40, 50, 60), some versions may include an instrument that is operable to apply two or more of monopolar RF, bipolar RF, or ultrasonic energy to tissue. In other words, two or more of such energy modalities may be incorporated into a single instrument. Examples of how such different modalities may be integrated into a single instrument are described in U.S. Pub. No. 2017/0202591, entitled "Modular Battery Powered Handheld Surgical Instrument with Selective Application of Energy Based on Tissue Characterization," published July 20, 2017. Other examples will be apparent to those skilled in the art in view of the teachings herein.

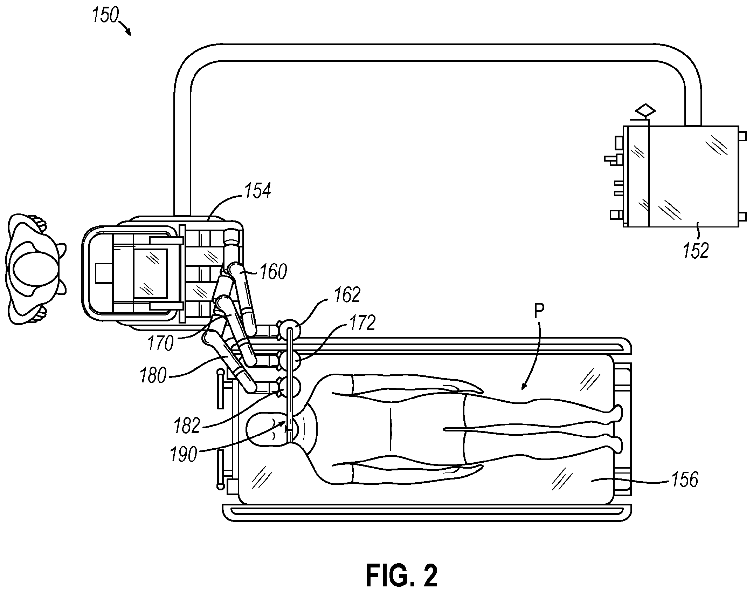

FIG. 2 shows an example of a robotic surgical system (150) in relation to a patient (P) on a table (156). System (150) of this example includes a control console (152) and a drive console (154). Console (152) is operable to receive user inputs from an operator; while drive console (154) is operable to convert those user inputs into motion of a set of robotic arms (160, 170, 180). In some versions, consoles (152, 154) collectively form an equivalent to console (20) described above. While consoles (152, 154) are shown as separate units in this example, consoles (152, 154) may in fact be combined as a single unit in some other examples.

Robotic arms (160, 170, 180) extend from drive console (154) in this example. In some other versions, robotic arms (160, 170, 180) are integrated into table (156) or some other structure. Each robotic arm (160, 170, 180) has a corresponding drive interface (162, 172, 182). In this example, three drive interfaces (162, 172, 182) are coupled with one single instrument assembly (190). In some other scenarios, each drive interface (162, 172, 182) is coupled with a separate respective instrument. By way of example only, a drive interface (162, 172, 182) may couple with a body of an instrument, like bodies (42, 52, 62) of instruments (40, 50, 60) described above. In any case, robotic arms (160, 170, 180) may be operable to move instrument (40, 50, 60, 190) in relation to the patient (P) and actuate any mechanically driven components of instrument (40, 50, 60, 190). Robotic arms (160, 170, 180) may also include features that provide a pathway for communication of electrical power to instrument (40, 50, 60, 190). For instance, cables (32, 34, 36) may be at least partially integrated into robotic arms (160, 170, 180). In some other versions, robotic arms (160, 170, 180) may include features to secure but not necessarily integrate cables (32, 34, 36). As yet another variation, cables (32, 34, 36) may simply stay separate from robotic arms (160, 170, 180). Other suitable features and arrangements that may be used to form robotic surgical systems (10, 150) will be apparent to those skilled in the art in view of the teachings herein.

In robotic surgical systems like robotic surgical systems (10, 150), each port (22, 24, 26, 28) may have a plurality of electrical features providing inputs and outputs between console (20, 152) and robotic arms (160, 170, 180) and/or instruments (40, 50, 60, 190). These electrical features may include sockets, pins, contacts, or various other features that are in close proximity with each other. In some scenarios, this proximity may provide a risk of power or signals undesirably crossing from one electrical feature to another electrical feature, which may cause equipment failure, equipment damage, sensor errors, and/or other undesirable results. In addition, or in the alternative, this proximity may provide a risk of generating electrical potentials between proximate components or creating capacitive couplings between electrical features. Such capacitive coupling may provide undesirable results such as power reductions, signal reductions, signal interference, patient injuries, and/or other undesirable results. It may therefore be desirable to provide features to prevent or otherwise address such occurrences at ports (22, 24, 26, 28).

Similarly, each robotic arm (160, 170, 180), each cable (32, 34, 36, 38), and/or each instrument (40, 50, 60, 190) may include a plurality of wires, traces in rigid or flexible circuits, and other electrical features that are in close proximity with each other. Such electrical features may also be in close proximity with other components that are not intended to provide pathways for electrical communication but are nevertheless formed of an electrically conductive material. Such electrically conductive mechanical features may include moving components (e.g., drive cables, drive bands, gears, etc.) or stationary components (e.g., chassis or frame members, etc.). This proximity may provide a risk of power or signals undesirably crossing from one electrical feature to another electrical feature and/or from one electrical feature to an electrically conductive mechanical feature, which may cause equipment failure, equipment damage, sensor errors, and/or other undesirable results. In addition, or in the alternative, this proximity may provide a risk of generating electrical potentials between proximate components or creating capacitive couplings between electrical features and/or between an electrical feature and an electrically conductive mechanical feature. Such capacitive coupling may provide undesirable results such as power reductions, signal reductions, signal interference, patient injuries, and/or other undesirable results. It may therefore be desirable to provide features to prevent or otherwise address such occurrences within robotic arms (160, 170, 180), within cables (32, 34, 36, 38), and/or within instruments (40, 50, 60, 190).

### II. Example of Handheld Surgical Instrument

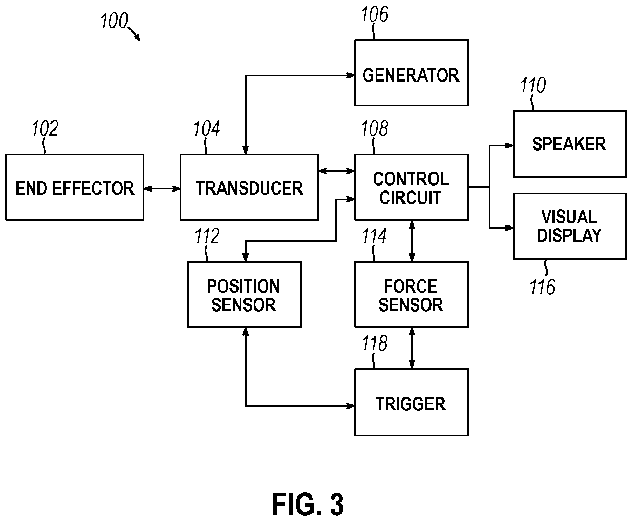

In some procedures, an operator may prefer to use a handheld surgical instrument in addition to, or in lieu of, using a robotic surgical system (10, 150). FIG. 3 illustrates an example of various components that may be integrated into a handheld surgical instrument (100). In addition to the following teachings, instrument (200) may be constructed and operable in accordance with at least some of the teachings of U.S. Pub. No. 2017/0202608, entitled "Modular Battery Powered Handheld Surgical Instrument Containing Elongated Multi-Layered Shaft," published July 20, 2017; and/or various other references cited herein. Instrument (100) of this example includes an end effector (102), an ultrasonic transducer (104), a power generator (106), a control circuit (108), a speaker (110), a position sensor (112), a force sensor (114), a visual display (116), and a trigger (118). In some versions, end effector (102) is disposed at a distal end of a shaft (not shown in FIG. 3), while the other components (104, 106, 108, 110, 112, 114, 116, 118) are incorporated into a handle assembly (not shown in FIG. 3) at the proximal end of the shaft. Some variations may also provide some of components (104, 106, 108, 110, 112, 114, 116, 118) in a separate piece of capital equipment. For instance, power generator (106), speaker (110), and/or visual display (116) may be incorporated into a separate piece of capital equipment that is coupled with instrument (100).

End effector (102) may be configured and operable like end effectors (46, 56, 66) described above, such that end effector (102) may be operable to apply monopolar RF energy, bipolar RF energy, or ultrasonic energy to tissue. Transducer (104) may be configured and operable like transducer (68). Generator (106) may be operable to provide electrical power as needed to drive transducer (68) and/or to provide RF energy via end effector (102). In versions where generator (106) is integrated into a handle assembly of instrument (106), generator (106) may comprise one or more battery cells, etc. Control circuit (108) may include one or more microprocessors and/or various other circuitry components that may be configured to provide signal processing and other electronic aspects of operability of instrument (100). Position sensor (112) may be configured to sense the position and/or orientation of instrument (102). In some versions, control circuit (108) is configured to vary the operability of instrument (102) based on data from position sensor (112). Force sensor (114) is operable to sense one or more force parameters associated with usage of instrument (100). Such force parameters may include force being applied to instrument (100) by the operator, force applied to tissue by end effector (102), or other force parameters as will be apparent to those skilled in the art in view of the teachings herein. In some versions, control circuit (108) is configured to vary the operability of instrument (102) based on data from force sensor (114). In some versions, one or both of sensors (112, 114) may be incorporated into end effector (102). In addition, or in the alternative, one or both of sensors (112, 114) may be incorporated into a shaft assembly (not shown) of instrument (100). Variations of instrument (100) may also incorporate various other kinds of sensors (e.g., in addition to or in lieu of sensors (112, 114) in end effector (102), in the shaft assembly, and/or elsewhere within instrument (100).

Trigger (118) is operable to control an aspect of operation of end effector (102), such as movement of a pivoting jaw, translation of a cutting blade, etc. Speaker (110) and visual display (116) are operable to provide audible and visual feedback to the operator relating to operation of instrument (100). The above-described components (102, 104, 106, 108, 110, 112, 114, 116, 118) of instrument (100) are illustrative examples, such that components (102, 104, 106, 108, 110, 112, 114, 116, 118) may be varied, substituted, supplemented, or omitted as desired.

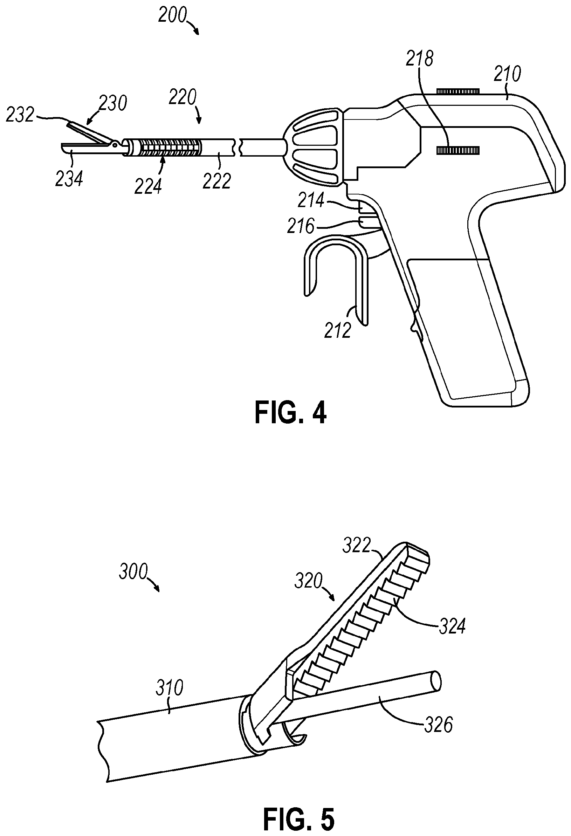

FIG. 4 shows an example of a form that instrument (100) may take. In particular, FIG. 4 shows a handheld instrument (200). In addition to the following teachings, instrument (200) may be constructed and operable in accordance with at least some of the teachings of U.S. Pub. No. 2017/0202591; and/or various other references cited herein. In the present example, instrument (200) includes a handle assembly (210), a shaft assembly (220), and an end effector (230). Handle assembly (210) includes a pivoting trigger (212), a first trigger button (214), a second trigger button (216), and an articulation control (218). Shaft assembly (220) includes a rigid shaft portion (222) and an articulation section (224). End effector (230) is distal to articulation section (224) and includes an upper jaw (232) and a lower jaw (234).

By way of example only, handle assembly (210) may include one or more of the above-described components (104, 106, 108, 110, 112, 114, 116, 118). Trigger (212) may be operable to drive upper jaw (232) to pivot toward lower jaw (234) (e.g., to grasp tissue between haws (232, 234)). Trigger buttons (214, 216) may be operable to activate delivery of energy (e.g., RF energy and/or ultrasonic energy) via end effector (230). Articulation control (218) is operable to drive deflection of shaft assembly (220) at articulation section (224), thereby driving lateral deflection of end effector (230) away from or toward the central longitudinal axis defined by rigid shaft portion (222). End effector (230) may include one or more electrodes that is/are operable to apply monopolar and/or bipolar RF energy to tissue. In addition, or in the alternative, end effector (230) may include an ultrasonic blade that is operable to apply ultrasonic energy to tissue. In some versions, end effector (230) is operable to apply two or more of monopolar RF energy, bipolar RF energy, or ultrasonic energy to tissue. Other suitable features and functionalities that may be incorporated into end effector (230) will be apparent to those skilled in the art in view of the teachings herein.

Instruments (150, 200) may include a plurality of wires, traces in rigid or flexible circuits, and other electrical features that are in close proximity with each other. Such electrical features may be located within handle assembly (210), within shaft assembly (220), and/or in end effector (230). Such electrical features may also be in close proximity with other components that are not intended to provide pathways for electrical communication but are nevertheless formed of an electrically conductive material. Such electrically conductive mechanical features may include moving components (e.g., drive cables, drive bands, gears, etc.) or stationary components (e.g., chassis or frame members, etc.). This proximity may provide a risk of power or signals undesirably crossing from one electrical feature to another electrical feature and/or from one electrical feature to an electrically conductive mechanical feature, which may cause equipment failure, equipment damage, sensor errors, patient injuries, and/or other undesirable results. In addition, or in the alternative, this proximity may provide a risk of generating electrical potentials between proximate components or creating capacitive couplings between electrical features and/or between an electrical feature and an electrically conductive mechanical feature. Such capacitive coupling may provide undesirable results such as power reductions, signal reductions, signal interference, and/or other undesirable results. It may therefore be desirable to provide features to prevent or otherwise address such occurrences within instruments (150, 200).

### III. Further Examples of Surgical Instrument Components

The following description relates to examples of different features that may be incorporated into any of the various instruments (40, 50, 60, 100, 190, 200) described above. While these examples are provided separate from each other, the features described in any of the following examples may be combined with the features described in other examples described below. Thus, the below-described features may be combined in various permutations as will be apparent to those skilled in the art in view of the teachings herein. Similarly, various ways in which the below-described features may be incorporated into any of the various instruments (40, 50, 60, 100, 190, 200) described above will be apparent to those skilled in the art in view of the teachings herein. The below-described features may be incorporated into robotically controlled surgical instruments (40, 50, 60, 190) and/or handheld surgical instruments (100, 200).

### A. Example of Ultrasonic End Effector

FIG. 5 shows a portion of an example of an ultrasonic instrument (300), including a shaft assembly (310) and an end effector (320). End effector (320) includes an upper jaw (322) and an ultrasonic blade (326). Upper jaw (322) is operable to pivot toward ultrasonic blade (326) to thereby compress tissue between a clamp pad (324) of upper jaw (322) and ultrasonic blade (326). When ultrasonic blade (326) is activated with ultrasonic vibrations, ultrasonic blade (326) may sever and seal tissue compressed against clamp pad (324). By way of example only, end effectors (66, 102, 230) may be configured and operable similar to end effector (320).

As noted above, instruments (150, 200) may include electrical features and/or electrically conductive mechanical features that may provide a risk of power or signals undesirably crossing from one electrical feature to another electrical feature and/or from one electrical feature to an electrically conductive mechanical feature. In addition, instruments (150, 200) may include electrical features and/or electrically conductive mechanical features that may provide a risk of generating electrical potentials between proximate components or creating capacitive couplings between electrical features and/or between an electrical feature and an electrically conductive mechanical feature. In the context of instrument (300), such risks may occur with respect to an acoustic waveguide in shaft assembly (310) leading to ultrasonic blade (326), as the acoustic waveguide may be formed of an electrically conductive material. In addition, instrument (300) may include one or more sensors in shaft assembly (310) and/or end effector (320); and may also include one or more electrodes and/or other electrical features in end effector (320). Other components of instrument (350) that may present the above-described risks will be apparent to those skilled in the art in view of the teachings herein.

### B. Example of Bipolar RF End Effector

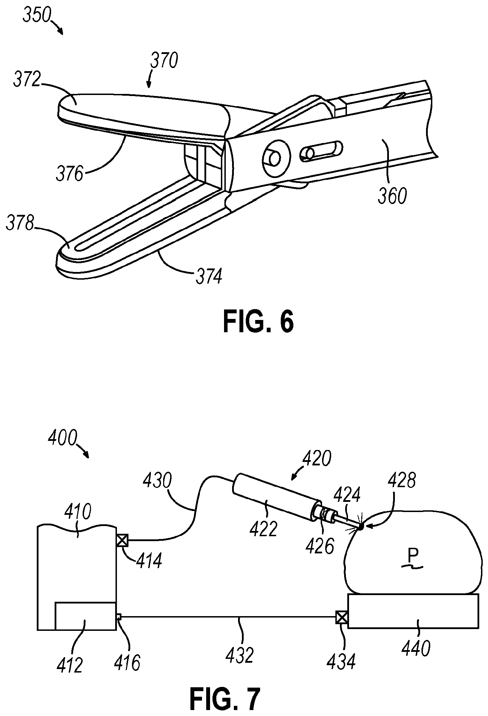

FIG. 6 shows a portion of an example of a bipolar RF instrument (350), including a shaft assembly (360) and an end effector (370). End effector (370) includes an upper jaw (372) and a lower jaw (374). Jaws (372, 374) are pivotable toward and away from each other. Upper jaw (372) includes a first electrode surface (376) while lower jaw (374) includes a second electrode surface (378). When tissue is compressed between jaws (372, 374), electrode surfaces (376, 378) may be activated with opposing polarities to thereby apply bipolar RF energy to the tissue. This bipolar RF energy may seal the compressed tissue. In some versions, end effector (370) further includes a translating knife member (not show) that is operable to sever tissue that is compressed between jaws (372, 374). Some variations of end effector (370) may also be operable to cooperate with a ground pad (e.g., ground pad (70)) to apply monopolar RF energy to tissue, such as by only activating one electrode surface (376, 378) or by activating both electrode surfaces (376, 378) at a single polarity. By way of example only, end effectors (64, 102, 230) may be configured and operable similar to end effector (370).

As noted above, instruments (150, 200) may include electrical features and/or electrically conductive mechanical features that may provide a risk of power or signals undesirably crossing from one electrical feature to another electrical feature and/or from one electrical feature to an electrically conductive mechanical feature. In addition, instruments (150, 200) may include electrical features and/or electrically conductive mechanical features that may provide a risk of generating electrical potentials between proximate components or creating capacitive couplings between electrical features and/or between an electrical feature and an electrically conductive mechanical feature. In the context of instrument (350), such risks may occur with respect to electrode surface (376, 378) and the wires or other electrical features that extend along shaft assembly (360) to reach electrode surfaces (376, 378). In addition, instrument (350) may include one or more sensors in shaft assembly (360) and/or end effector (370); and may also include one or more electrodes and/or other electrical features in end effector (370). Other components of instrument (350) that may present the above-described risks will be apparent to those skilled in the art in view of the teachings herein.

### C. Example of Monopolar Surgical Instrument Features

FIG. 7 shows an example of a monopolar RF energy delivery system (400) that includes a power generator (410), a delivery instrument (420), and a ground pad assembly (440). In addition to the following teachings, instrument (420) may be constructed and operable in accordance with at least some of the teachings of U.S. Pub. No. 2019/0201077; and/or various other references cited herein. Power generator (410) may be operable to deliver monopolar RF energy to instrument (420) via a cable (430), which is coupled with power generator (410) via a port (414). In some versions, port (414) includes an integral sensor. By way of example only, such a sensor in port (414) may be configured to monitor whether excess or inductive energy is radiating from power generator (410) and/or other characteristics of energy being delivered from power generator (410) via port (414). Instrument (420) includes a body (422), a shaft (424), a sensor (426), and a distal electrode (428) that is configured to contact a patient (P) and thereby apply monopolar RF energy to the patient (P). By way of example only, sensor (426) may be configured to monitor whether excess or inductive energy is radiating from instrument (420). Based on signals from sensor (426), a control module in power generator (410) may passively throttle the ground return from ground pad assembly (440) based on data from sensor (426).

In some versions, ground pad assembly (440) comprises one or more resistive continuity ground pads that provide direct contact between the skin of the patient (P) and one or more metallic components of the ground pad. In some other versions, ground pad assembly (440) comprises a capacitive coupling ground pad that includes a gel material that is interposed between the patient (P) and the ground return plate. In the present example, ground pad assembly (440) is positioned under the patient (P) and is coupled to power generator (410) via a cable (432) via ports (416, 434). Either or both of ports (416, 434) may include an integral sensor. By way of example only, such a sensor in either or both of ports (416, 434) may be configured to monitor whether excess or inductive energy is radiating from ground pad assembly (440).

As noted above, instruments (150, 200) may include electrical features and/or electrically conductive mechanical features that may provide a risk of power or signals undesirably crossing from one electrical feature to another electrical feature and/or from one electrical feature to an electrically conductive mechanical feature. In addition, instruments (150, 200) may include electrical features and/or electrically conductive mechanical features that may provide a risk of generating electrical potentials between proximate components or creating capacitive couplings between electrical features and/or between an electrical feature and an electrically conductive mechanical feature. In the context of instrument (420), such risks may occur with respect to sensor (426), distal electrode (428), and/or any other electrical components in instrument (420). Other components of instrument (420) that may present the above-described risks will be apparent to those skilled in the art in view of the teachings herein. Such risks may be greater in versions instrument (420) that are dedicated to providing monopolar RF energy than in the context of bipolar RF instruments such as instrument (350) because a dedicated monopolar RF instrument may lack a ground return path that might otherwise prevent or mitigate the above risks.

### D. Example of Articulation Section in Shaft Assembly

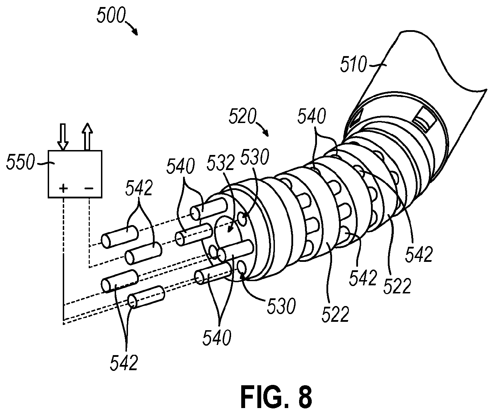

FIG. 8 illustrates a portion of an instrument (500) that includes a shaft (510) with an articulation section (520). In addition to the following teachings, instrument (500) may be constructed and operable in accordance with at least some of the teachings of U.S. Pub. No. 2017/0202591; and/or various other references cited herein. In the present example, an end effector (550) is positioned at the distal end of articulation section (520). Articulation section (520) includes a plurality of segments (522) and is operable to laterally deflect end effector (550) away from and toward the central longitudinal axis of shaft (510). A plurality of wires (540) extend through shaft (510) and along articulation section (520) to reach end effector (550) and thereby deliver electrical power to end effector (550). By way of example only, end effector (550) may be operable to deliver monopolar and/or bipolar RF energy to tissue as described herein. A plurality of push-pull cables (542) also extend through articulation section (520). Push-pull cables (542) may be coupled with an actuator (e.g., similar to articulation control (218)) to drive articulation of articulation section (520). Segments (522) are configured to maintain separation between, and provide structural support to, wires (540) and push-pull cables (542) along the length of articulation section (520). Articulation section (520) of this example also defines a central passageway (532). By way of example only, central passageway (532) may accommodate an acoustic waveguide (e.g., in variations where end effector (550) further includes an ultrasonic blade), may provide a path for fluid communication, or may serve any other suitable purpose. Alternatively, central passageway (532) may be omitted.

As noted above, instruments (150, 200) may include electrical features and/or electrically conductive mechanical features that may provide a risk of power or signals undesirably crossing from one electrical feature to another electrical feature and/or from one electrical feature to an electrically conductive mechanical feature. In addition, instruments (150, 200) may include electrical features and/or electrically conductive mechanical features that may provide a risk of generating electrical potentials between proximate components or creating capacitive couplings between electrical features and/or between an electrical feature and an electrically conductive mechanical feature. In the context of instrument (500), such risks may occur with respect to wires (540) and/or push-pull cables (542). In addition, instrument (500) may include one or more sensors in shaft assembly (510) and/or end effector (550); and may also include one or more electrodes and/or other electrical features in end effector (550). Other components of instrument (500) that may present the above-described risks will be apparent to those skilled in the art in view of the teachings herein.

### E. Example of Wiring to End Effector

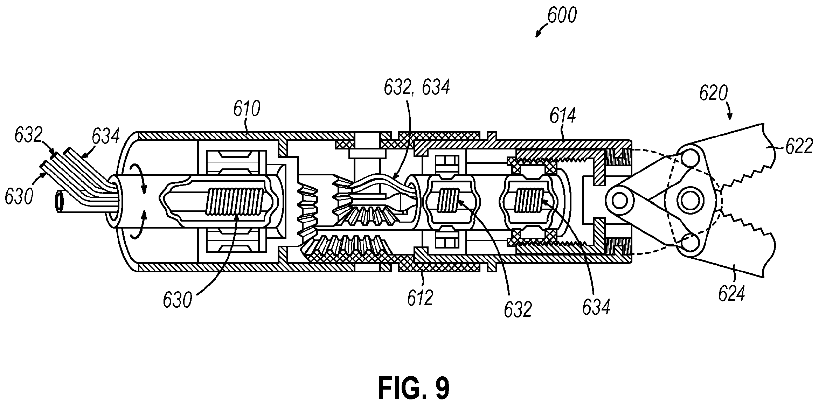

FIG. 9 illustrates a portion of an instrument (600) that includes a shaft (610) with n first articulating segment (612) and a second articulating segment (614). In addition to the following teachings, instrument (600) may be constructed and operable in accordance with at least some of the teachings of U.S. Pub. No. 2017/0202605, entitled "Modular Battery Powered Handheld Surgical Instrument and Methods Therefor," published July 20, 2017; and/or various other references cited herein. In the present example, end effector (620) is positioned at the distal end of second articulating segment (614). End effector (620) of this example includes a pair of jaws (622, 624) that are operable to pivot toward and away from each other to grasp tissue. In some versions, one or both of jaws (622, 624) includes one or more electrodes that is/are operable to apply RF energy to tissue as described herein. In addition, or in the alternative, end effector (620) may include an ultrasonic blade and/or various other features. Segments (612, 614) may be operable to pivot relative to shaft (610) and relative to each other to thereby deflect end effector (620) laterally away from or toward the central longitudinal axis of shaft (610).

Instrument (600) of this example further includes a first wire set (630) spanning through shaft (610), a second wire set (632) spanning through shaft (610) and both segments (612, 614), and a third wire set (634) spanning further through shaft (610) and both segments (612, 614). Wire sets (630, 632, 634) may be operable to control movement of segments (612, 614) relative to shaft (610). For instance, power may be communicated along one or more of wire sets (630, 632, 634) to selectively engage or disengage corresponding clutching mechanisms, to thereby allow lateral deflection of one or both of segments (612, 614) relative to shaft (610); and or rotation of one or both of segments (612, 614) relative to shaft (610). Alternatively, power may be communicated along one or more of wire sets (630, 632, 634) to drive corresponding solenoids, motors, or other features to actively drive lateral deflection of one or both of segments (612, 614) relative to shaft (610); and or rotation of one or both of segments (612, 614) relative to shaft (610). In versions where end effector (620) is operable to apply RF energy to tissue, one or more additional wires may extend along shaft (610) and segments (612, 614), in addition to wire sets (630, 632, 634).

As noted above, instruments (150, 200) may include electrical features and/or electrically conductive mechanical features that may provide a risk of power or signals undesirably crossing from one electrical feature to another electrical feature and/or from one electrical feature to an electrically conductive mechanical feature. In addition, instruments (150, 200) may include electrical features and/or electrically conductive mechanical features that may provide a risk of generating electrical potentials between proximate components or creating capacitive couplings between electrical features and/or between an electrical feature and an electrically conductive mechanical feature. In the context of instrument (600), such risks may occur with respect to wire sets (630, 632, 634), the electrical components that wire sets (630, 632, 634) are coupled with, and/or other features that drive lateral deflection of one or both of segments (612, 614) relative to shaft (610). In addition, instrument (600) may include one or more sensors in shaft assembly (610) and/or end effector (620); and may also include one or more electrodes and/or other electrical features in end effector (620). Other components of instrument (600) that may present the above-described risks will be apparent to those skilled in the art in view of the teachings herein.

### F. Example of Sensors in Shaft Assembly

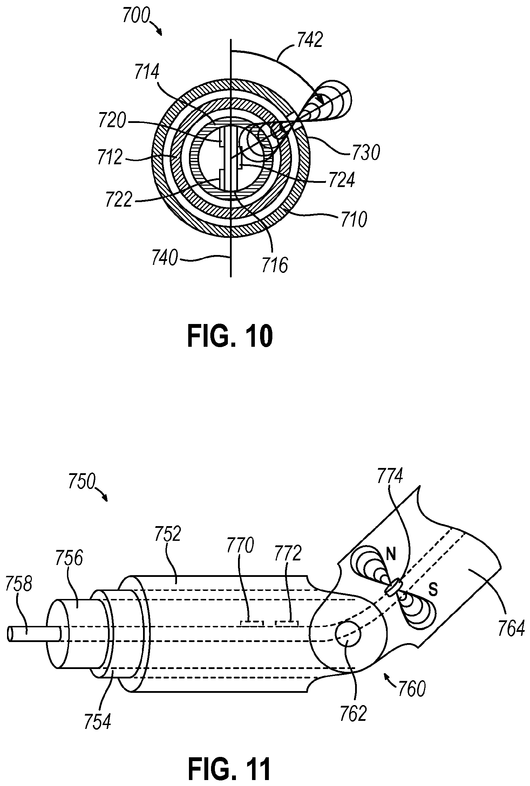

FIG. 10 shows an example of another shaft assembly (700) that may be incorporated into any of the various instruments (40, 50, 60, 100, 190, 200, 300, 350, 400, 500, 600) described herein. In addition to the following teachings, shaft assembly (700) may be constructed and operable in accordance with at least some of the teachings of U.S. Pub. No. 2017/0202608; and/or various other references cited herein. Shaft assembly (700) of this example includes an outer shaft (710), a first inner shaft (712), and a second inner shaft (714). A support member (716) spans diametrically across the interior of second inner shaft (714). By way of example only, support member (716) may comprise a circuit board, a flex-circuit, and/or various other electrical components. A plurality of sensors (720, 722, 724) are positioned on support member (716) in the present example. A magnet (730) is embedded in outer shaft (710) which is operable to rotate about inner shafts (712, 714).

In some versions, rotation of outer shaft (710) about inner shafts (712, 714) drives rotation of an end effector (not shown), located at the distal end of shaft assembly (700), about a longitudinal axis of shaft assembly (700). In some other versions, rotation of outer shaft (710) about inner shafts (712, 714) drives lateral deflection of the end effector away from or toward the longitudinal axis of shaft assembly (700). Alternatively, rotation of outer shaft (710) about inner shafts (712, 714) may provide any other results. In any case, sensors (720, 722, 724) may be configured to track the position of magnet (730) and thereby determine a rotational position (742) of outer shaft (710) relative to a fixed axis (740). Thus, sensors (720, 722, 724) may collectively serve as a position sensor like position sensor (112) of instrument (100).

FIG. 11 shows an example of another shaft assembly (750) that may be incorporated into any of the various instruments (40, 50, 60, 100, 190, 200, 300, 350, 400, 500, 600) described herein. In addition to the following teachings, shaft assembly (750) may be constructed and operable in accordance with at least some of the teachings of U.S. Pub. No. 2017/0202608; and/or various other references cited herein. Shaft assembly (750) of this example includes a plurality of coaxially positioned proximal shaft segments (752, 754, 756) and a distal shaft segment (764). Distal shaft segment (764) is pivotably coupled with proximal shaft segment (752) via a pin (762) to form an articulation joint (760). An end effector (not shown) may be positioned distal to distal shaft segment (764), such that articulation joint (760) may be utilized to deflect the end effector laterally away from or toward a central longitudinal axis defined by proximal shaft segments (752, 754, 756). A flex circuit (758) spans along shaft segments (752, 754, 756, 764) and is operable to flex as shaft assembly (750) bends at articulation joint (760).

A pair of sensors (770, 772) are positioned along flex circuit (758) within the region that is proximal to articulation joint (760); while a magnet (774) is positioned on flex circuit (758) (or elsewhere within distal shaft segment (764)) in the region that is distal to articulation joint (760). Magnet (774) thus moves with distal shaft segment (764) as distal shaft segment (764) pivots relative to proximal shaft segments (752, 754, 756) at articulation joint (760); while sensors (770, 772) remain stationary during such pivoting. Sensors (770, 772) are configured to track the position of magnet (774) and thereby determine a pivotal position of distal shaft segment (764) relative to proximal shaft segments (752, 754, 756). In other words, sensors (770, 772) and magnet (774) cooperate to enable determination of the articulation bend angle formed by shaft assembly (750). Thus, sensors (770, 772) may collectively serve as a position sensor like position sensor (112) of instrument (100).

As noted above, instruments (150, 200) may include electrical features and/or electrically conductive mechanical features that may provide a risk of power or signals undesirably crossing from one electrical feature to another electrical feature and/or from one electrical feature to an electrically conductive mechanical feature. In addition, instruments (150, 200) may include electrical features and/or electrically conductive mechanical features that may provide a risk of generating electrical potentials between proximate components or creating capacitive couplings between electrical features and/or between an electrical feature and an electrically conductive mechanical feature. In the context of instruments (700, 750), such risks may occur with respect to sensors (720, 722, 724, 770, 772), the electrical components that sensors (720, 722, 724, 770, 772) are coupled with, and/or other features within the shaft assemblies of instruments (700, 750). Other components of instruments (700, 750) that may present the above-described risks will be apparent to those skilled in the art in view of the teachings herein.

### G. Example of Drive Controls in Body and Shaft Assembly of Instrument

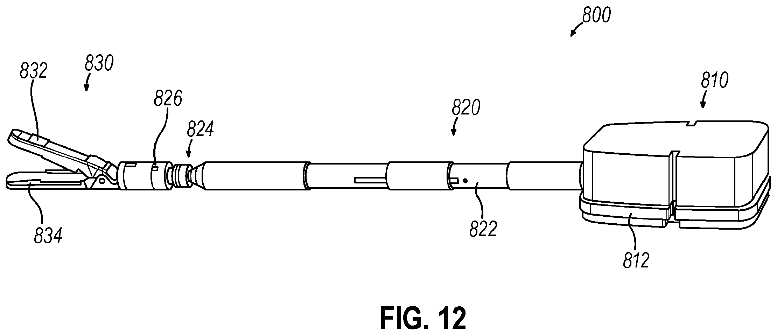

FIGS. 12-14 show an example of an instrument (800) that may be incorporated into a robotic surgical system, such as the robotic surgical systems (10, 150) described herein. In addition to the following teachings, instrument (800) may be constructed and operable in accordance with at least some of the teachings of U.S. Pat. No. 9,125,662; and/or various other references cited herein. Instrument (800) of this example includes a body (810), a shaft assembly (820), and an end effector (830). Body (810) includes a base (812) that is configured to couple with a complementary component of a robotic arm (e.g., one of robotic arms (160, 170, 180)). Shaft assembly (820) includes a rigid proximal portion (822), an articulation section (824), and a distal portion (826). End effector (830) is secured to distal portion (826). Articulation section (824) is operable to deflect distal portion (826) and end effector (830) laterally away from and toward the central longitudinal axis defined by proximal portion (822). End effector (830) of this example includes a pair of jaws (832, 834). By way of example only, end effector (830) may be configured and operable like any of the various end effectors (46, 56, 66, 102, 230, 320, 350, 620) described herein.

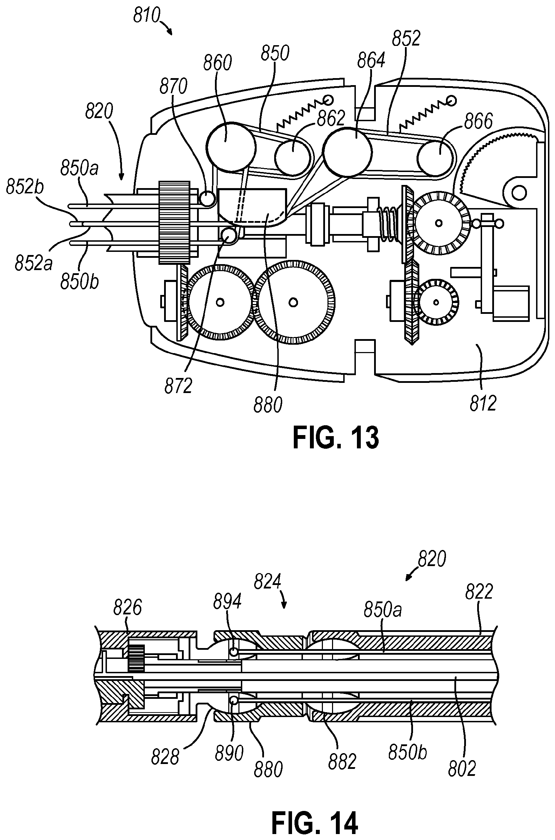

As shown in FIGS. 13-14, a plurality of drive cables (850, 852) extend from body (810) to articulation section (824) to drive articulation of articulation section (824). Cable (850) is wrapped around a drive pulley (862) and a tensioner (860). Cable (850) further extends around a pair of guides (870, 872), such that cable (850) extends along shaft assembly (820) in two segments (850a, 850b). Cable (852) is wrapped around a drive pulley (866) and a tensioner (864). Cable (852) further extends around a guide (880), such that cable (852) extends along shaft assembly (820) in two segments (852a, 852b). In the present example, each drive pulley (862, 866) is configured to couple with a corresponding drive member (e.g., drive spindle, etc.) of the component of the robotic arm to which base (812) is secured. When drive pulley (862) is rotated, one segment (850a) of cable (850) will translate in a first longitudinal direction along shaft assembly (820); while the other segment (850b) will simultaneously translate in a second (opposite) direction along shaft assembly (820). Similarly, when drive pulley (866) is rotated, one segment (852a) of cable (852) will translate in a first longitudinal direction along shaft assembly (820); while the other segment (852b) will simultaneously translate in a second (opposite) direction along shaft assembly (820).

As shown in FIG. 14, articulation section (824) of the present example includes an intermediate shaft segment (880) that is longitudinally interposed between proximal portion (822) and distal portion (826). A ball feature (828) at the proximal end of distal portion (826) is seated in a socket at the distal end of intermediate shaft segment (880), such that distal portion (826) is operable to pivot relative to intermediate shaft segment (880) along one or more planes. Segments (850a, 850b) of drive cable (850) terminate in corresponding ball-ends (894, 890), which are secured to ball feature (828) of distal portion (822). Drive cable (850) is thus operable to drive pivotal movement of distal portion (826) relative to intermediate shaft segment (880) based on the direction in which drive pulley (862) rotates. A ball feature (882) at the proximal end of intermediate portion (880) is seated in a socket at the distal end of proximal portion (822), such that intermediate portion (880) is operable to pivot relative to proximal portion (822) along one or more planes. In some versions, this pivotal movement of intermediate portion (880) relative to proximal portion (822) is driven by cable (852). As also shown in FIG. 14, an electrical cable (802) passes through articulation section (824). Electrical cable (802) provides a path for electrical communication to end effector (830), thereby allowing for delivery of electrical power (e.g., RF energy) to one or more electrodes in end effector (830), providing a path for electrical signals from one or more sensors in end effector (830) to be communicated back to body (810), and/or other forms of electrical communication.

As noted above, instruments (150, 200) may include electrical features and/or electrically conductive mechanical features that may provide a risk of power or signals undesirably crossing from one electrical feature to another electrical feature and/or from one electrical feature to an electrically conductive mechanical feature. In addition, instruments (150, 200) may include electrical features and/or electrically conductive mechanical features that may provide a risk of generating electrical potentials between proximate components or creating capacitive couplings between electrical features and/or between an electrical feature and an electrically conductive mechanical feature. In the context of instrument (800), such risks may occur with respect to drive cables (850, 852), the components that (850, 852) are coupled with, electrical features within shaft assembly (820), and/or other features within instrument (800). Other components of instrument (800) that may present the above-described risks will be apparent to those skilled in the art in view of the teachings herein.

### H. Example of Electrical Features at Interface between Modular Components of Instrument

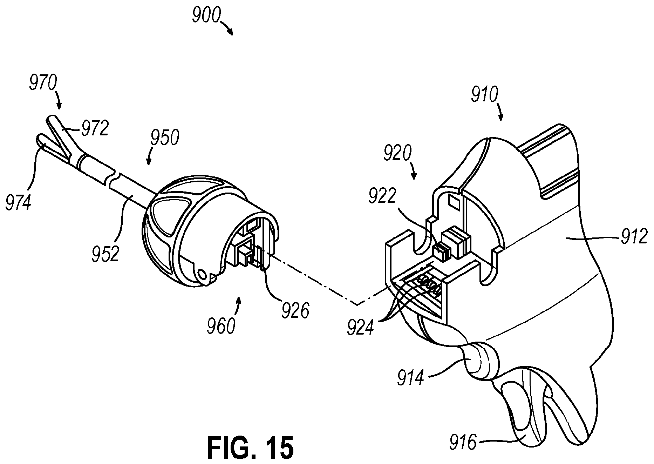

In some instances, it may be desirable to provide a surgical instrument that allows for modular coupling and decoupling of components. For instance, FIG. 15 shows an example of an instrument (900) that includes a handle assembly (910) and a modular shaft assembly (950). While instrument (900) of this example is handheld, similar features and modularity may be readily incorporated into a robotically controlled instrument. Handle assembly (910) of this example includes a body (912), an activation button (914), a pivoting trigger (916), and a shaft interface assembly (920). Shaft interface assembly (920) includes a mechanical drive feature (922) and an array of electrical contacts (924). Electrical contacts (924) may be in electrical communication with a control circuit, power source, and/or various other electrical features within handle assembly (910) as will be apparent to those skilled in the art in view of the teachings herein.

Shaft assembly (950) includes a shaft section (952) and an end effector (970), which includes a pair of jaws (972, 874). Shaft section (952) and end effector (970) may be configured and operable in accordance with any of the various shaft assemblies and end effectors described herein. Shaft assembly (950) of this example further includes a handle interface assembly (960). Handle interface assembly (960) includes a mechanical drive feature (962) and a plurality of electrical contacts (not shown). These electrical contacts of handle interface assembly (960) may be in electrical communication with one or more electrodes, sensors, and/or other electrical components within shaft section (952) and/or end effector (970) as will be apparent to those skilled in the art in view of the teachings herein.

When shaft assembly (950) is coupled with handle assembly (910), mechanical drive feature (922) of handle assembly (910) mechanically couples with mechanical drive feature (962) of shaft assembly (950), such that mechanical drive features (922, 962) may cooperate to communicate motion from a motive power source in handle assembly (910) (e.g., pivoting trigger (916), a motor, etc.) to one or more components within shaft section (952) and, in some versions, end effector (970). In some versions, mechanical drive features (922, 962) cooperate to communicate rotary motion from a motive power source in handle assembly (910) (e.g., pivoting trigger (916), a motor, etc.) to one or more components within shaft section (952) and, in some versions, end effector (970). In addition, or in the alternative, mechanical drive features (922, 962) may cooperate to communicate linear translational motion from a motive power source in handle assembly (910) (e.g., pivoting trigger (916), a motor, etc.) to one or more components within shaft section (952) and, in some versions, end effector (970).

When shaft assembly (950) is coupled with handle assembly (910), electrical contacts (924) of shaft interface assembly (920) also couple with complementary electrical contacts of handle interface assembly (960), such that these contacts establish continuity with each other and thereby enable the communication of electrical power, signals, etc. between handle assembly (910) and shaft assembly (950). In addition to or in lieu of having contacts (924), electrical continuity may be provided between handle assembly (910) and shaft assembly (950) via one or more electrical couplings at mechanical drive features (922, 962). Such electrical couplings may include slip couplings and/or various other kinds of couplings as will be apparent to those skilled in the art in view of the teachings herein.

In some scenarios where electrical power or electrical signals are communicated across mating contacts that provide electrical continuity between two components of an instrument (e.g., contacts (924) of shaft interface assembly (920) and complementary electrical contacts of handle interface assembly (960)), there may be a risk of short circuits forming between such contacts. This may be a particular risk when contacts that are supposed to be electrically isolated from each other are located in close proximity with each other, and the area in which these contacts are located may be exposed to fluids during use of the instrument. Such fluid may create electrical bridges between contacts and/or bleed signals that are being communicated between contacts that are supposed to be coupled with each other. It may therefore be desirable to provide features to prevent or otherwise address such occurrences at contacts of an instrument like instrument (900).

In some scenarios where electrical power or electrical signals are communicated across mechanical couplings between different components of an instrument (e.g., via slip couplings, etc.), such couplings might provide variable electrical resistance in a shaft assembly or other assembly of the instrument. For instance, motion at mechanical drive features (922, 962) may provide variable electrical resistance at an electrical slip coupling between mechanical drive features (922, 962); and this variable electrical resistance may impact the communication of electrical power or electrical signals across the slip coupling. This may in turn result in signal loss or power reductions. It may therefore be desirable to provide features to prevent or otherwise address such occurrences at electrical couplings that are found at mechanical couplings between two moving parts of an instrument like instrument (900).

### IV. Examples of Electrosurgical System Shaft Voltage Monitoring Features

The following description relates to examples of different features that may be incorporated into any of the various surgical systems described above. Thus, the below-described features may be combined in various permutations as will be apparent to those skilled in the art in view of the teachings herein. Similarly, various ways in which the below-described features may be incorporated into any of the various surgical systems described above will be apparent to those skilled in the art in view of the teachings herein. It should be understood that the below-described features may be incorporated into robotically controlled surgical instruments and/or handheld surgical instruments.

Some versions of the instruments described herein may provide a floating ground with respect to conductive components within a shaft assembly of the instrument. In scenarios where a floating ground exists, such conductive components are not electrically coupled with earth ground. A floating ground may isolate ground return paths within the instrument and bring them to one point, effectively creating an ad hoc ground that is isolated relative to actual ground. A floating ground may have an associated ad hoc voltage, and control circuitry may adjust a voltage associated with the floating ground. Ultimately, a floating ground may provide electrical isolation for components within an electrical circuit in the absence of earth ground. Electrically conductive components may be understood as having a floating potential or voltage when such electrically conductive components are not electrically coupled with earth ground.

Some aspects of the present disclosure are presented for monitoring voltage potentials in components of a shaft assembly, and adaptively adjusting power, adjusting sensing signals, and/or providing some other kind of system response based on detected voltage potentials in components of the shaft assembly. The voltage potentials of one shaft component may be monitored relative to the common return path and relative to the potentials of the other shaft components. In some cases, variations in voltage potentials present at different components of an electrosurgical system may cause ground loops. For instance, the difference in potential between the return path ground on the generator and the local ground on the end effector that is in use may cause a ground loop. The effects of ground loops may depend on the severity of the potential difference between the grounding points. Small ground loops may inject noise onto a system and cause interruption or loss of communication on data lines. Large ground loops may cause damage to electronic components or cause the entire system to reset or become temporarily inoperative. It may therefore be desirable to actively monitor variations in potentials and to take corrective action.

As will be described in greater detail below, variations in potentials or voltages may be monitored to control the electrical connection to the components in order for undesired voltages to be drained or floated relative to the return path, based on a comparison of the measured voltage potential to a predetermined max threshold value. In some instances, shaft components and/or control electronics may be shifted between an electrically floating condition and an interconnected condition on an intermittent basis. Such shifting may ensure accurate local measuring by sensors and accurate operation by active electrical components; while allowing the draining of otherwise parasitic power signals and/or preventing incidental unintended electrification of system components. In some instances, local sensing may be paused or adjusted while a voltage is drained as part of a safety draining process as described herein. Corrective action may include any one or more of adjusting the noise correction thresholds, adjusting the transformation to correct for the introduced error, providing the system a "blackout" where it needs to ignore the sensors within the effects of the potential shift, or even rebooting or shutting off power to the sensor to protect it from damage.

In some versions, monitoring for variations in potential may include monitoring the shaft components for voltage potentials relative to one another and/or relative to the return path to the generator. For example, in versions where the shaft assembly is comprised of a plurality of metallic components, the instrument may include a wiring harness or flex circuit to connect the end effector to the outer housing assembly.

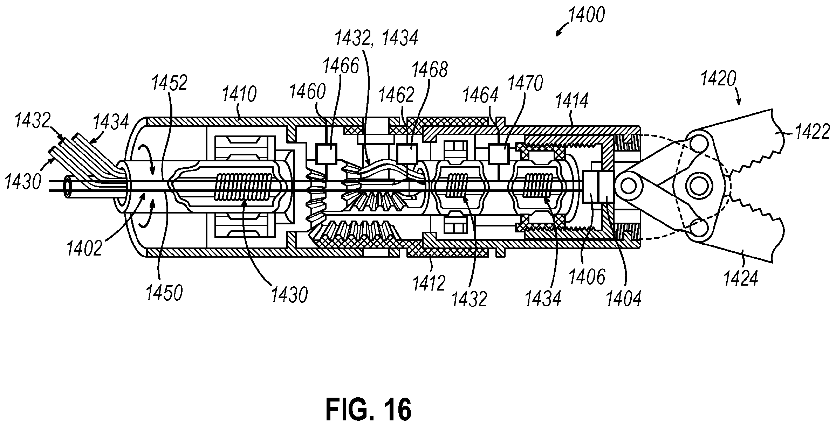

FIG. 16 illustrates a portion of an instrument (1400) that includes an elongated shaft (1410). It should be understood that, while instrument (1400) is illustrated and described in detail, various other electrosurgical instruments have been contemplated including, but not limited to, the instruments described herein above. A console (not shown) of instrument (1400) may receive voltage measurements from one or more sensors, as will be described below, and react accordingly to initiate a corrective action. By way of example only, the console may be configured similar to console (20) described above with reference to FIG. 1, and may include a data processor configured and operable to initiate the corrective action, adjust the power profile sent to instrument (1400), or float or drain any of the components forming the body of instrument (1400). Further, the console may be a component of a robotic electrosurgical system, as described above. Various suitable forms that a console for instrument (1400) may take will be apparent to those skilled in the art in view of the teachings herein.

Instrument (1400) of the present example is substantially similar to instrument (600) of FIG. 9, as described above, except for the differences described below. Instrument (1400) includes a first articulating segment (1412) and a second articulating segment (1414). End effector (1420) is positioned at the distal end of second articulating segment (1414). End effector (1420) of this example includes a pair of jaws (1422, 1424) that are operable to pivot toward and away from each other to grasp tissue. In some versions, one or both of jaws (1422, 1424) includes one or more electrodes that is/are operable to apply RF energy to tissue as described herein. Such electrodes may be powered via electrical connectors (1404, 1406), which are routed through instrument via a wiring harness (1402). While a wiring harness (1402) is used in the present example, any other suitable kind of conductor assembly (e.g., flex circuit ribbon, etc.) may be used as will be apparent to those skilled in the art in view of the teachings herein. In addition, or in the alternative, end effector (1420) may include an ultrasonic blade and/or various other features in addition to, or in lieu of, including jaws (1422, 1424). Segments (1412, 1414) may be operable to pivot relative to shaft (1410) and relative to each other to thereby deflect end effector (1420) laterally away from or toward the central longitudinal axis of shaft (1410).

Instrument (1400) of this example further includes a first wire set (1430) spanning through shaft (1410), a second wire set (1432) spanning through shaft (1410) and both segments (1412, 1414), and a third wire set (1434) spanning further through shaft (1410) and both segments (1412, 1414). Wire sets (1430, 1432, 1434) may be operable to control movement of segments (1412, 1414) relative to shaft (1410). For instance, power may be communicated along one or more of wire sets (1430, 1432, 1434) to selectively engage or disengage corresponding clutching mechanisms, to thereby allow lateral deflection of one or both of segments (1412, 1414) relative to shaft (1410); and or rotation of one or both of segments (1412, 1414) relative to shaft (1410). Alternatively, power may be communicated along one or more of wire sets (1430, 1432, 1434) to drive corresponding solenoids, motors, or other features to actively drive lateral deflection of one or both of segments (1412, 1414) relative to shaft (1410); and or rotation of one or both of segments (1412, 1414) relative to shaft (1410). In versions where end effector (1420) is operable to apply RF energy to tissue, one or more additional wire sets, such as wiring harness (1402) extends along shaft (1410) and segments (1412, 1414), in addition to wire sets (1430, 1432, 1434), to couple with connectors (1404, 1406) to provide power to end effector (1420).

Connectors (1404, 1406) of the present example include a proximal connector (1404) and a distal connector (1406), which are configured to removably mate with each other. Wiring harness (1402) is coupled with proximal connector (1406), such that wires (1450, 1542) of wiring harness (1402) couple with distal connector (1404), which is then configured to mate with proximal connector (1406) to provide power to end effector (1420). By way of example only, such power may include bipolar RF energy for electrodes on end effector (1420). Return path ground (1452) (e.g., ground wire, ground trace, etc.) from end effector (1402) may have intermediate electrical connections to metallic components in the shaft assembly, such as shaft (1410), first articulating segment (1412), and second articulating segment (1414), in order to be able to monitor the voltage potential of each component (1410, 1412, 1414) relative to return path (1452) and relative to each other component (1410, 1412, 1414). This monitoring may be used by the console to control the electrical connection to the components (1410, 1412, 1414) in order to allow them to be electrically drained or floated relative to return path (1452) based on the comparison of the measured voltage potential to a predetermined max threshold voltage value.

As shown, wiring harness (1402), or alternatively, a flexible circuit, connects end-effector (1420) to a handle or other body of the electrosurgical instrument (1400) and may include conductive attachment points (1460, 1462, 1464) to conductive structures within the instrument (1400). These conductive attachment locations (1460, 1462, 1464) may allow integrated sensors (1466, 1468, 1470) to monitor the voltage potential of the components (1410, 1412, 1414), respectively, as each relates to the control electronics (e.g., the generator or related components) and return path ground (1452). While sensors (1466, 1468, 1470) are integrated adjacent to corresponding attachment locations (1460, 1462, 1464) in the present example, other configurations may be used. By way of example only, a wire, conductive trace, or other electrically conductive path may extend from each attachment location (1460, 1462, 1464) to a proximal location (e.g., to a proximal portion of shaft (1410), to a body of instrument (1400) that is proximal to shaft (1410), to a console that is coupled with instrument (1400), etc.); and be coupled with return path (1452) at such a proximal location in order to effectively monitor potentials between attachment locations (1460, 1462, 1464) and return path (1452).

If the system detects a potential change in one of the components (1410, 1412, 1414), the system may determine if the potential change exists due to an externally applied voltage source or from a capacitive coupling of the component (1410, 1412, 1414) with another component (1410, 1412, 1414) that is being intentionally activated with electrical power. Once the system determines the source of the voltage variation, the system may actively ground or clamp off the voltage potential, warn the user of external contact with another energized instrument, and/or apply an adjustment to the rest of the sensors (1466, 1468, 1470) proportionate to the effect caused by the one sensed potential. In some versions, sensors (1466, 1468, 1470) include high impedance sensors positioned between the metallic frame component (1410, 1412, 1414) and return path (1452). In versions utilizing a flexible circuit in place of wiring harness (1404), wires (1450, 1542) may instead be included as conductive traces routed through the body of instrument (1400).

In some versions, sensors (1466, 1468, 1470) are configured to monitor the voltage potential relative ground path (1452) of all the metallic shaft components, such as components (1410, 1412, 1414), and only selectively ground the ones that have accumulated electrical current to remove. Thus, to operate in the safest configuration, each component (1410, 1412, 1414) may remain electrically floating unless a particular component (1410, 1412, 1414) requires discharge. In this context, "electrically floating" means that a component (1410, 1412, 1414) is not electrically coupled with ground. In some scenarios, a component (1410, 1412, 1414) that is electrically floating may have a certain floating voltage. Such floating voltages may be induced by electromagnetic fields that are generated in component (1410, 1412, 1414) by proximate, activated components. Such floating voltages may also be caused by charge accumulating within component (1410, 1412, 1414).

In some versions, each component (1410, 1412, 1414) may be shifted from an electrically floated configuration to an interconnected configuration, where one or more components (1410, 1412, 1414) are at least temporarily electrically coupled together. This may be done intermittently to ensure accurate local measuring and operation while still allowing draining of any parasitic or incidental electrifying of the system. Thus, each component (1410, 1412, 1414) may be maintained in an electrically floating state by default; and only be grounded through the console in the event that it is determined that a particular component (1410, 1412, 1414) has built up a potential that exceeds the threshold value such that the component (1410, 1412, 1414) should be discharged.

As previously noted, shaft (1410) and/or end effector (1420) may include one or more operation sensors operable to sense one or more parameters associated with operation of end effector (1420). By way of example only, such an operation sensor may include a force sensor (e.g., force sensor (114), etc.) that is operable to sense a clamping force that is being applied by jaws (1422, 1424) to tissue; or a force sensor that is operable to sense transverse loads being applied to shaft (1410) during engagement of tissue by end effector (1420). By way of further example only, an operation sensor may include a temperature sensor that is operable to sense the temperature of end effector (1420) or the temperature of tissue that is being engaged by end effector (1420). As another merely illustrative example, an operation sensor may include an impedance sensor that is operable to sense the impedance of tissue that is being engaged by end effector (1420). As yet another merely illustrative example, an operation sensor may include a position sensor (e.g., position sensor (112), sensors (720, 722, 724), sensors (770, 772), etc.) that is operable to sense a position or orientation of shaft (1410) and/or end effector (1420). Other kinds of operation sensors that may be incorporated into shaft (1410) and/or end effector (1420) will be apparent to those skilled in the art in view of the teachings herein.

In versions of instrument (1400) with operation sensors such as those described above, the voltage shifting described herein may ensure accurate local measuring by such operation sensors and reduce noise that might otherwise occur within signals from such operation sensors. In some instances, sensing by operation sensors may be paused or adjusted while a voltage is drained as part of a safety draining process as described herein. Corrective action may also include any one or more of adjusting noise correction thresholds, adjusting the transformation to correct for the introduced error, providing the system a "blackout" where console needs to ignore signals from operation sensors within the effects of the potential shift, or even rebooting or shutting off power to an operation sensor to protect the operation sensor from damage.

In some versions, sensors (1466, 1468, 1470) and/or any other sensors within shaft (1410) or end effector (1420) may be paused, or otherwise deactivated and disconnected, while one or more of components (1410, 1412, 1414) is at least temporarily grounded, whether such grounding connection is made via another component (1410, 1412, 1414), via dedicated ground path (1452), or via any other already- grounded component of instrument (1400).

In some instruments, such as a bipolar RF surgical stapling instrument with an end effector having bipolar electrodes near surgical staples, the bipolar electrodes may run the risk of contacting surgical staples, thereby creating a short circuit between the bipolar electrodes via one or more surgical staples. In monopolar instruments, capacitive coupling currents may accumulate on any metallic components forming the instrument shaft. To alleviate the risks associates with these scenarios, plastic components (or "metal insert interruptions") may be included within the shaft assembly to avoid having a shaft that is metallic along its entire length. For example, an electrically insulating member may be included in one or more of components (1410, 1412, 1414) to minimize the impact of these electrical current risks on the surrounding components; and further to minimize the propagation of the capacitive couple current upstream and downstream of instrument (1400). A molded plastic member, or otherwise a non-conductive member, may be inserted where the metal portions of components (1410, 1412, 1414) overlap. In such versions, the shaft assembly may lack a continuous path for unintended electrical continuity along the full length of the shaft assembly (other than such paths as intentionally provided by wires, etc.). In other words, electrically conductive structural components of the shaft assembly, that are not intended to conduct electricity, may include non-conductive structural components interposed therebetween to provide interruptions disrupting the electrical continuity that might otherwise exist. In some versions, adjacent components (1410, 1412, 1414) may include holes or keying features that allow one long, non-conductive plate to be coupled to the other via interlocking injection molded plastic cross-sections. Other suitable ways in which electrically interruptive, non-conductive structural components may be integrated into a shaft assembly will be apparent to those skilled in the art in view of the teachings herein.

### VI. Miscellaneous

Versions of the devices described above may have application in conventional medical treatments and procedures conducted by a medical professional, as well as application in robotic-assisted medical treatments and procedures.

It should be understood that any of the versions of instruments described herein may include various other features in addition to or in lieu of those described above. By way of example only, any of the instruments described herein may also include one or more of the various features disclosed in any of the various references. It should also be understood that the teachings herein may be readily applied to any of the instruments described in any of the other references cited herein, such that the teachings herein may be readily combined with the teachings of any of the references cited herein in numerous ways. Other types of instruments into which the teachings herein may be incorporated will be apparent to those of ordinary skill in the art.

In addition to the foregoing, the teachings herein may be readily combined with the teachings of U.S. Pat. App. No. 17/136137, entitled "Filter for Monopolar Surgical Instrument Energy Path," filed on even date herewith. Various suitable ways in which the teachings herein may be combined with the teachings of U.S. Pat. App. No. 17/136137 will be apparent to those of ordinary skill in the art in view of the teachings herein.

In addition to the foregoing, the teachings herein may be readily combined with the teachings of U.S. Pat. App. No. 17/136139, entitled "Electrosurgical Instrument System with Parasitic Energy Loss Monitor,". Various suitable ways in which the teachings herein may be combined with the teachings of U.S. Pat. App. No. 17/136139 will be apparent to those of ordinary skill in the art in view of the teachings herein.

In addition to the foregoing, the teachings herein may be readily combined with the teachings of U.S. Pat. App. No. 17/136141, entitled "Energized Surgical Instrument System with Multi-Generator Output Monitoring," . Various suitable ways in which the teachings herein may be combined with the teachings of U.S. Pat. App. No. 17/136141 will be apparent to those of ordinary skill in the art in view of the teachings herein.

In addition to the foregoing, the teachings herein may be readily combined with the teachings of U.S. Pat. App. No. 17/136154, entitled "Electrosurgical Instrument with Electrical Resistance Monitor at Rotary Coupling,". Various suitable ways in which the teachings herein may be combined with the teachings of U.S. Pat. App. No. 17/136154 will be apparent to those of ordinary skill in the art in view of the teachings herein.

In addition to the foregoing, the teachings herein may be readily combined with the teachings of U.S. Pat. App. No. 17/136158, entitled "Electrosurgical Instrument with Modular Component Contact Monitoring,". Various suitable ways in which the teachings herein may be combined with the teachings of U.S. Pat. App. No. 17/136158 will be apparent to those of ordinary skill in the art in view of the teachings herein.

It should also be understood that any ranges of values referred to herein should be read to include the upper and lower boundaries of such ranges. For instance, a range expressed as ranging "between approximately 1.0 inches and approximately 1.5 inches" should be read to include approximately 1.0 inches and approximately 1.5 inches, in addition to including the values between those upper and lower boundaries.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by an operator immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam. Having shown and described various embodiments of the present invention, further adaptations of the methods and systems described herein may be accomplished by appropriate modifications by one of ordinary skill in the art without departing from the disclosure. Several of such potential modifications have been mentioned, and others will be apparent to those skilled in the art. For instance, the examples, embodiments, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A surgical instrument (1400), comprising:
(a) a shaft assembly (1410) having a plurality of conductive components (1410, 1412, 1414), wherein each conductive component of the plurality of conductive components is configured with a floating voltage;
(b) an end effector (1420) positioned at a distal end of the shaft assembly, wherein the end effector is operable to apply energy to tissue of a patient;
(c) a console (20) operable to power the end effector;
(d) a conductor assembly (1402) disposed within the shaft assembly and configured to transfer power from the console to the end effector, wherein the conductor assembly includes a ground return path (1452); and
(e) a plurality of voltage sensors (1466, 1468, 1470), wherein each conductive component of the plurality of conductive components is configured to couple with a corresponding voltage sensor of the plurality of voltage sensors and with the ground return path, wherein the plurality of voltage sensors are operable to measure a voltage potential difference of the coupled conductive component relative to a ground potential defined by the ground return path;
wherein the console is configured to initiate a corrective action based on the measured voltage potential difference.

2. The surgical instrument of claim 1, wherein initiating a corrective action based on the measured voltage potential difference comprises:
(i) determining whether the measured voltage potential difference exceeds a maximum threshold value, and
(ii) when the measured voltage potential difference exceeds the maximum threshold value, initiate the corrective action.

3. The surgical instrument of claim 1 or 2, wherein the shaft assembly or the end effector further comprises an operation sensor (112, 720, 770) operable to sense a parameter associated with operation of the end effector, wherein the corrective action includes disregarding a signal from the operation sensor.

4. The surgical instrument of claim 1 or 2, wherein the shaft assembly or the end effector further comprises an operation sensor operable to sense a parameter associated with operation of the end effector, wherein the corrective action includes disconnecting power to the operation sensor.

5. The surgical instrument of claim 1 or 2, wherein the shaft assembly or the end effector further comprises an operation sensor operable to sense a parameter associated with operation of the end effector, the corrective action includes rebooting the operation sensor.

6. The surgical instrument of claim 1 or 2, wherein the corrective action includes discharging a selected conductive component of the plurality of conductive components to the ground return path.

7. The surgical instrument of claim 6, wherein the corrective action further includes deactivating sensing from the voltage sensor associated with the selected conductive component while the voltage is being discharged.

8. The surgical instrument of claim 1 or 2, wherein each of the plurality of conductive components is configured to be electrically interconnected with one another, wherein the conductive components share a common voltage potential upon being electrically interconnected.

9. The surgical instrument of claim 8, wherein the console is operable to reduce the common voltage potential relative to the ground potential from the conductive components while the conductive components are electrically interconnected.

10. The surgical instrument of claim 1 or 2, wherein the plurality of voltage sensors comprise high impedance voltage sensors.

11. The surgical instrument of claim 1 or 2, wherein the console includes a generator configured to provide RF energy to the end effector.

12. The surgical instrument of claim 1 or 2, wherein the conductor assembly comprises a wiring harness.

13. The surgical instrument of claim 1 or 2, wherein the console is a component of a robotic electrosurgical system (150).

14. The surgical instrument of claim 1, further comprising an operation sensor operable to sense a parameter associated with operation of the end effector, wherein the corrective action includes adjusting an electrical noise correction threshold associated with the operation sensor.

15. The surgical instrument of claim 1, further comprising an operation sensor operable to sense a parameter associated with operation of the end effector, wherein the corrective action includes adjusting a voltage transformation associated with the operation sensor.

## Patentansprüche

1. Chirurgisches Instrument (1400), umfassend:
(a) eine Wellenanordnung (1410) mit einer Vielzahl von leitfähigen Komponenten (1410, 1412, 1414), wobei jede leitfähige Komponente der Vielzahl von leitfähigen Komponenten mit einer Schwebespannung konfiguriert ist;
(b) einen Endeffektor (1420), der an einem distalen Ende der Wellenanordnung positioniert ist, wobei der Endeffektor betriebsfähig ist, um Energie an Gewebe eines Patienten anzulegen;
(c) eine Konsole (20), die betriebsfähig ist, um den Endeffektor mit Energie zu versorgen;
(d) eine Leiteranordnung (1402), die innerhalb der Wellenanordnung angeordnet und dazu konfiguriert ist, Energie von der Konsole zu dem Endeffektor zu übertragen, wobei die Leiteranordnung einen Erdrückleitungspfad (1452) einschließt; und
e) eine Vielzahl von Spannungssensoren (1466, 1468, 1470), wobei jede leitende Komponente der Vielzahl von leitenden Komponenten dazu konfiguriert ist, mit einem entsprechenden Spannungssensor der Vielzahl von Spannungssensoren und mit dem Erdrückleitungspfad gekoppelt zu werden, wobei die Vielzahl von Spannungssensoren betriebsfähig ist, eine Spannungspotentialdifferenz der gekoppelten leitfähigen Komponente relativ zu einem durch den Erdrückleitungspfad definierten Erdpotential zu messen;
wobei die Konsole so konfiguriert ist, dass sie auf Grundlage der gemessenen Spannungspotentialdifferenz eine Korrekturmaßnahme einleitet.

2. Chirurgisches Instrument nach Anspruch 1, wobei das Einleiten einer Korrekturmaßnahme auf der Grundlage der gemessenen Spannungspotentialdifferenz Folgendes umfasst:
(i) Bestimmen, ob die gemessene Spannungspotentialdifferenz einen maximalen Schwellenwert überschreitet, und
(ii) wenn die gemessene Spannungspotenzialdifferenz den maximalen Schwellenwert überschreitet, Einleiten der Korrekturmaßnahme.

3. Chirurgisches Instrument nach Anspruch 1 oder 2, wobei die Wellenanordnung oder der Endeffektor ferner einen Betriebssensor (112, 720, 770) umfasst, der betriebsfähig ist, um einen dem Betrieb des Endeffektors zugeordneten Parameter zu erfassen, wobei die Korrekturmaßnahme das Nichtbeachten eines Signals von dem Betriebssensor einschließt.

4. Chirurgisches Instrument nach Anspruch 1 oder 2, wobei die Wellenanordnung oder der Endeffektor ferner einen Betriebssensor umfasst, der betriebsfähig ist, um einen dem Betrieb des Endeffektors zugeordneten Parameter zu erfassen, wobei die Korrekturmaßnahme das Unterbrechen der Stromversorgung des Betriebssensors einschließt.

5. Chirurgisches Instrument nach Anspruch 1 oder 2, wobei die Wellenanordnung oder der Endeffektor ferner einen Betriebssensor umfasst, der betriebsfähig ist, um einen dem Betrieb des Endeffektors zugeordneten Parameter zu erfassen, wobei die Korrekturmaßnahme das Neustarten des Betriebssensors einschließt.

6. Chirurgisches Instrument nach Anspruch 1 oder 2, wobei die Korrekturmaßnahme das Entladen einer ausgewählten leitfähigen Komponente der Vielzahl von leitfähigen Komponenten zum Erdungsrückleitungspfad einschließt.

7. Chirurgisches Instrument nach Anspruch 6, wobei die Korrekturmaßnahme ferner das Deaktivieren der Erfassung durch den Spannungssensor einschließt, der der ausgewählten leitfähigen Komponente zugeordnet ist, während die Spannung entladen wird.

8. Chirurgisches Instrument nach Anspruch 1 oder 2, wobei jede der Vielzahl von leitfähigen Komponenten so konfiguriert ist, dass sie elektrisch miteinander verbunden werden, wobei die leitfähigen Komponenten ein gemeinsames Spannungspotential aufweisen, wenn sie elektrisch miteinander verbunden sind.

9. Chirurgisches Instrument nach Anspruch 8, wobei die Konsole so betriebsfähig ist, dass sie das gemeinsame Spannungspotential der leitfähigen Komponenten gegenüber dem Erdpotential reduziert, während die leitfähigen Komponenten elektrisch miteinander verbunden sind.

10. Chirurgisches Instrument nach Anspruch 1 oder 2, wobei die Vielzahl von Spannungssensoren Spannungssensoren mit hoher Impedanz umfasst.

11. Chirurgisches Instrument nach Anspruch 1 oder 2, wobei die Konsole einen Generator einschließt, der dazu konfiguriert ist, dem Endeffektor HF-Energie bereitzustellen.

12. Chirurgisches Instrument nach Anspruch 1 oder 2, wobei die Leiteranordnung einen Kabelbaum umfasst.

13. Chirurgisches Instrument nach Anspruch 1 oder 2, wobei die Konsole eine Komponente eines robotergestützten elektrochirurgischen Systems (150) ist.

14. Chirurgisches Instrument nach Anspruch 1, das ferner einen Betriebssensor umfasst, der betriebsfähig ist, um einen dem Betrieb des Endeffektors zugeordneten Parameter zu erfassen, wobei die Korrekturmaßnahme das Anpassen eines dem Betriebssensor zugeordneten Korrekturschwellenwerts für elektrisches Rauschen einschließt.

15. Chirurgisches Instrument nach Anspruch 1, das ferner einen Betriebssensor umfasst, der betriebsfähig ist, um einen dem Betrieb des Endeffektors zugeordneten Parameter zu erfassen, wobei die Korrekturmaßnahme das Anpassen einer dem Betriebssensor zugeordneten Spannungstransformation einschließt.

## Revendications

1. Instrument chirurgical (1400), comprenant :
(a) un ensemble arbre (1410) ayant une pluralité de composants conducteurs (1410, 1412, 1414), dans lequel chaque composant conducteur de la pluralité de composants conducteurs est configuré avec une tension flottante ;
(b) un effecteur terminal (1420) positionné au niveau d'une extrémité distale de l'ensemble arbre, dans lequel l'effecteur terminal peut être actionné pour appliquer de l'énergie à un tissu d'un patient ;
(c) une console (20) pouvant être actionnée pour alimenter l'effecteur terminal ;
(d) un ensemble conducteur (1402) disposé à l'intérieur de l'ensemble arbre et configuré pour transférer l'énergie de la console à l'effecteur terminal, dans lequel l'ensemble conducteur comporte une voie de retour à la terre (1452) ; et
(e) une pluralité de capteurs de tension (1466, 1468, 1470), dans lequel chaque composant conducteur de la pluralité de composants conducteurs est configuré pour se coupler avec un capteur de tension correspondant de la pluralité de capteurs de tension et avec la voie de retour à la terre, dans lequel la pluralité de capteurs de tension peut être actionnée pour mesurer une différence de potentiel de tension du composant conducteur couplé par rapport à un potentiel de terre défini par la voie de retour à la terre ;
dans lequel la console est configurée pour lancer une action corrective sur la base de la différence de potentiel de tension mesurée.

2. Instrument chirurgical selon la revendication 1, dans lequel le lancement d'une action corrective sur la base de la différence de potentiel de tension mesurée comprend :
(i) le fait de déterminer si la différence de potentiel de tension mesurée dépasse une valeur seuil maximale, et
(ii) lorsque la différence de potentiel de tension mesurée dépasse la valeur seuil maximale, le lancement de l'action corrective.

3. Instrument chirurgical selon la revendication 1 ou 2, dans lequel l'ensemble arbre ou l'effecteur terminal comprend en outre un capteur de fonctionnement (112, 720, 770) capable de détecter un paramètre associé au fonctionnement de l'effecteur terminal, dans lequel l'action corrective comporte le fait d'ignorer un signal provenant du capteur de fonctionnement.

4. Instrument chirurgical selon la revendication 1 ou 2, dans lequel l'ensemble arbre ou l'effecteur terminal comprend en outre un capteur de fonctionnement pouvant être actionné pour détecter un paramètre associé au fonctionnement de l'effecteur terminal, dans lequel l'action corrective comporte la déconnexion de l'alimentation du capteur de fonctionnement.

5. Instrument chirurgical selon la revendication 1 ou 2, dans lequel l'ensemble arbre ou l'effecteur terminal comprend en outre un capteur de fonctionnement pouvant être actionné pour détecter un paramètre associé au fonctionnement de l'effecteur terminal, l'action corrective comporte le redémarrage du capteur de fonctionnement.

6. Instrument chirurgical selon la revendication 1 ou 2, dans lequel l'action corrective comporte la décharge d'un composant conducteur sélectionné de la pluralité de composants conducteurs vers la voie de retour à la terre.

7. Instrument chirurgical selon la revendication 6, dans lequel l'action corrective comporte en outre la désactivation de la détection du capteur de tension associé au composant conducteur sélectionné pendant que la tension est en cours de décharge.

8. Instrument chirurgical selon la revendication 1 ou 2, dans lequel chacun de la pluralité de composants conducteurs est configuré pour être interconnecté électriquement avec un autre, dans lequel les composants conducteurs partagent un potentiel de tension commun lors de l'interconnexion électrique.

9. Instrument chirurgical selon la revendication 8, dans lequel la console peut être actionnée pour réduire le potentiel de tension commun par rapport au potentiel de la terre des composants conducteurs pendant que les composants conducteurs sont électriquement interconnectés.

10. Instrument chirurgical selon la revendication 1 ou 2, dans lequel la pluralité de capteurs de tension comprend des capteurs de tension à haute impédance.

11. Instrument chirurgical selon la revendication 1 ou 2, dans lequel la console comporte un générateur configuré pour fournir de l'énergie RF à l'effecteur terminal.

12. Instrument chirurgical selon la revendication 1 ou 2, dans lequel l'ensemble conducteur comprend un faisceau de câbles.

13. Système selon la revendication 1 ou 2, dans lequel la console est un composant d'un système électrochirugical robotique (150).

14. Instrument chirurgical selon la revendication 1, comprenant en outre un capteur de fonctionnement capable de détecter un paramètre associé au fonctionnement de l'effecteur terminal, dans lequel l'action corrective comporte l'ajustement d'un seuil de correction de bruit électrique associé au capteur de fonctionnement.

15. Instrument chirurgical selon la revendication 1, comprenant en outre un capteur de fonctionnement pouvant être actionné pour détecter un paramètre associé au fonctionnement de l'effecteur terminal, dans lequel l'action corrective comporte l'ajustement d'une transformation de tension associée au capteur de fonctionnement.
